# EUROPEAN PATENT APPLICATION

(11) **EP 1 972 268 A1**
(43) Date of publication of application: **24.09.2008**
(21) Application number: 07005837.5
(22) Date of filing: 21.03.2007
(51) Int. Cl.: A61B 5/00, G06F 17/00

(54) **Method of compressing electrocardiogram data and electrocardiogram telemetry system using the same**

(71) Applicant: Nihon Kohden Corporation, Shinjuku-ku Tokyo (JP)
(72) Inventor: Fujihashi, Hisayuki, Shinjuku-ku, Tokyo (JP); Suzuki, Katsuyoshi, Shinjuku-ku, Tokyo (JP); Tone, Katsuhide, Shinjuku-ku, Tokyo (JP); Inai, Takashi, Shinjuku-ku, Tokyo (JP)
(74) Representative: Grünecker, Kinkeldey, Stockmair & Schwanhäusser Anwaltssozietät

(57) **Abstract**

In an electrocardiogram telemeter, a data acquirer is operable to acquire electrocardiogram data. A data compressor Is operable to compress the electrocardiogram data with either a wavelet transform, a Huffman coding, or an arithmetic coding, thereby generating compressed electrocardiogram data adapted to be transmitted to a remote receiver which is configured to reconstruct the electrocardiogram data.

## Description

### BACKGROUND

The present invention relates to a method of compressing electrocardiogram data and an electrocardiogram telemetry system configured to be capable of effectively compressing and transmitting monitoring data of a biological signals, such as an electrocardiogram (hereinafter, referred to as ECG), of a plurality of patients, receiving the compressed data, and appropriately reconstructing the compressed data as ECG data.

ECG telemetry systems are being used as systems capable of comprehensively and effectively manage a plurality of patients for medical purposes, for example, diagnosis of an abnormal ECG, such as arhythmia or angina in the daily life of each patient, evaluation of the effect of medicine, such as an antiarhythmia agent or an antianginal agent, evaluation of pacemaker treatment, and expectation of ischemic heart disease prognosis,

A system processing a large amount of ECG data which requires data compression in order to record digital data of ECG data in a 24 hour period has been known as a related art. For example, as an ECG data compression, it is considered that an ECG is repetition of heart beats having considerably similar shapes and an amount of information required for the heart beats is large. In this method, a template of the waveform of a heart beat of an ECG is subtracted from an ECG waveform and the residual data (signals) are calculated such that redundancy of the data is eliminated, and the signal is compressed by, for example, a known straight-line approximation or coding.

However, in the related-art data compression, the compression efficiency is lowered. For this reason, Japanese Patent Publication No. 6-237909A proposes an electrocardiogram data processor which can compress data with high compression rate and high precision by reducing the information amount of residual data for which pulse removal is performed by using a multi-template corresponding to an average waveform of normal pulses for a prescribed time period when ST measurement is performed or normal and abnormal pulses generated by a learning function.

Further, Japanese Patent Publication No. 9-224917A proposes a Holter electrocardiograph provided with an infrared radiation communication device capable of communicating efficiently compressed data to an external equipment according to a prescribed procedure. In this Holter electrocardiograph, an ECG input from electrodes is amplified by an amplifier, is quantized by an A/D converter, and is input to a digital signal processor. Next, the digitalized ECG input to the digital signal process is wavelet-transformed and is then compressed with high efficiency. The ECG data is stored in a flash memory. Then, the ECG data stored in the Holter electrocardiograph is input to the external equipment through an infrared interface unit.

In the above medical telemetry systems, an amount of ECG data capable of being transmitted is small. For this reason, when it is required ECG data having a number of vector cardiograms larger than a number of vector cardiograms capable of being transmitted, ECG data having a required number of vector cardiograms is estimated by numerical calculation on the basis of ECG data received by a reception side. As described above, since the number of vector cardiograms of ECG capable of being transmitted is small, it is difficult to sufficiently transmit ECG information to accurately take biological information of a patient. Further, when ECG having required vector cardiograms is estimated on the basis of transmittable ECG data, a result different from real biological information may be obtained.

From this view point, as described above, various techniques for compressing ECG data has been proposed but any specific technique for a compression appropriate to ECG data has not been proposed. Therefore, the above-mentioned techniques are not suitable for practical use and the structure of systems may become complicated.

### SUMMARY

It is therefore one advantageous aspect of the invention to provide a method of compressing ECG data and an ECG telemetry system using the same, in which multiplexing of transmittable ECG data is made possible, by properly and efficiently perform ECG data compression, in order to improve telemeter performance.

According to one aspect of the invention, there is provided a method of compressing an electrocardiogram data, comprising:
acquiring the electrocardiogram data;
subjecting the acquired electrocardiogram data to a frequency filtering; and
compressing the electrocardiogram data, which has been subjected to a frequency filtering, with either a wavelet transform, a Huffman coding, or an arithmetic coding.

The wavelet transform may include dividing the electrocardiogram data into a plurality of data pieces which are respectively associated with different frequency ranges. The wavelet transform may include at least one of: deleting at least one of the divided pieces; deleting at least a part of at least one of the divided pieces, in which noises having a wave height less than a prescribed value are superposed; and deleting at least a part of at least one of the divided pieces, in which noises having a wave height no less than the prescribed value are superposed.

According to one aspect of the invention, there is provided an electrocardiogram telemeter, comprising:
a data acquirer, operable to acquire electrocardiogram data;
a filter, operable to subject the electrocardiogram data acquired by the data acquirer to a frequency filtering; and
a data compressor, operable to compress the electrocardiogram data, which has been subjected to the frequency filtering, with either a wavelet transform, a Huffman coding, or an arithmetic coding, thereby generating compressed electrocardiogram data adapted to be transmitted to a remote receiver which is configured to reconstruct the electrocardiogram data.

The data compressor may be operable to divide the electrocardiogram data into a plurality of data pieces which are respectively associated with different frequency ranges. The data compressor may be operable to perform the wavelet transform by at least one of:
deleting at least one of the divided pieces;
deleting at least a part of at least one of the divided pieces, in which noises having a wave height less than a prescribed value are superposed; and
deleting at least a part of at least one of the divided pieces, in which noises having a wave height no less than the prescribed value are superposed.

By deleting data unnecessary for the electrocardiogram and data having no effect on the characteristics of the electrocardiogram on the basis of the frequency of occurrence of each data piece and compressing the remaining biological information data so that a required minimum amount of data is selected from electrocardiogram data and compressed, it is possible to make multiplexing of transmittable electrocardiogram data and properly and efficiently perform data compression of the electrocardiogram data.

In a case where the wavelet transform is performed, it is possible to properly and efficiently perform electrocardiogram data compression by dividing the electrocardiogram data into high-frequency components and low-frequency components, and deleting data having frequency components unnecessary for exhibit characteristics as electrocardiogram data or data having negligible effect on the characteristics so as to reduce the amount of data.

Since the electrocardiogram data can be properly and efficiently compressed and multiplexed, it is possible to improve the performance of the electrocardiogram telemeter.

### BRIEF DESCRIPTION OF THE DRAWINGS

Fig. 1 is a block diagram showing an ECG telemetry system according to one embodiment of the invention.
Fig. 2 is a flow chart showing an ECG data compression executed by a data compressor in the ECG telemetry system.
Fig. 3 is a diagram for explaining wavelet transforms performed in the ECG data compression.
Fig. 4 is a diagram for explaining data reconstruction performed in a receiver in the ECG telemetry system.
Fig. 5 is a diagram showing a relationship between a frequency range and the number of times that the wavelet transform is applied to the data.
Fig. 6 is a diagram showing a relationship between the amount of data and the frequency range when the wavelet transform is applied to 64 original data pieces five times.
Fig. 7A shows an ECG waveform on which noise has been superposed.
Fig. 7B shows a reconstructed waveform in a case where the data compression is performed with respect to the first half of the ECG waveform.
Fig. 7C shows a reconstructed waveform in a case where the data compression is performed with respect to the second half of the ECG waveform.

### DETAILED DESCRIPTION OF EXEMPLARY EMBODIMENTS

Exemplary embodiments of the invention will be described below In detail with reference to the accompanying drawings.

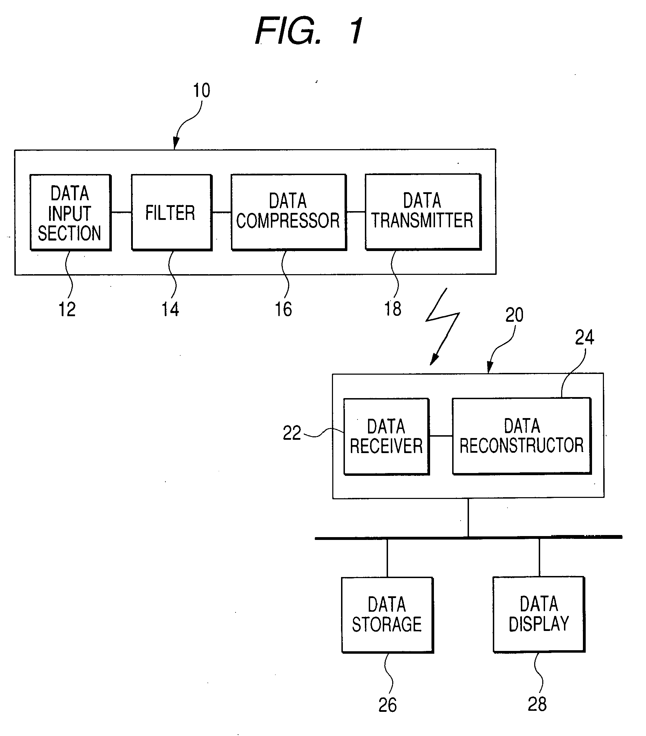

As shown In Fig. 1, an ECG telemetry system according to one embodiment of the invention comprises a transmitter 10 which includes an ECG data input section 12 to which an ECG signal detected from a living body is input, a filter 14 filtering ECG data having been inputted into the ECG data input section 12, a data compressor 16 compressing the ECG data having been filtered by the filter 14 by using a method (which will be described below), and a data transmitter 18 transmitting the ECG data having been compressed by the ECG data compressor 16.

In this system, a receiver 20 includes a data receiver 22 receiving the ECG data transmitted by the data transmitter 18 of the transmitter 10, and a data reconstructer 24 reconstructing the compressed ECG data received by the data receiver 22. While the ECG data received by the receiver 20 is stored in a data storage 26, the ECG data are reconstructed by the data reconstructer 24 and then displayed on a data display 28. Alternatively, the ECG data received by the receiver 20 may be reconstructed by the data reconstructer 24 and is then displayed on the data display 28 without being stored in the data storage 26. In this case, the data storage 26 may be omitted from the system.

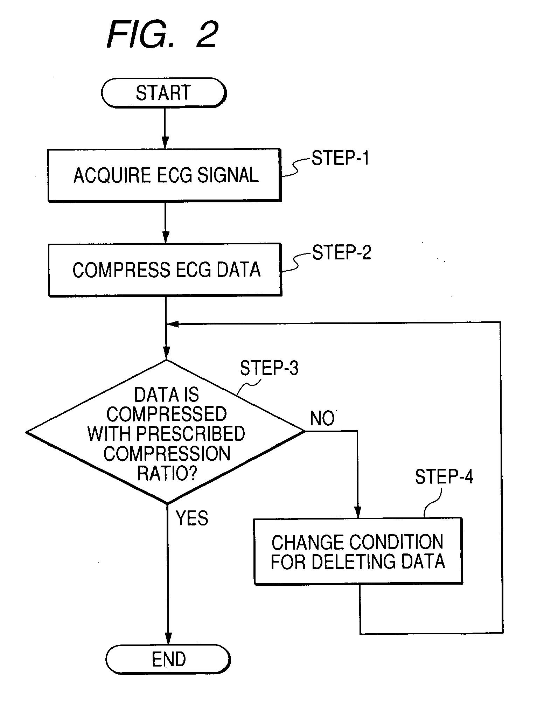

Next, the ECG data compression used by the data compressor 16 of the ECG telemetry system will be described with reference to Fig. 2.

First, the data compressor 16 acquires the ECG data having been filtered by the filter 14 (STEP-1). The filter 14 performs a low-pass filtering on raw ECG data such that the ECG data are converted into data capable of easily being compressed by the data compressor 16. The filter 14 may perform a digital low-pass filtering using, for example, 40 Hz as a cutoff frequency on data on which an analog low-pass filtering using, for example, 150 Hz as a cutoff frequency has been performed. The data that is filtered by the above-mentioned method can be used, as original data used to acquire ECG difference data.

The ECG data having filtered as described above are compressed (STEP-2). An ECG data compression used in this case will be described below. In a case where a data compression using Huffman coding or arithmetic coding is performed on the ECG data, a required minimum amount of data is selected from the ECG data on the basis of the frequency of occurrence of each data piece and is then compressed. In a case where a data compression using wavelet transform is performed, biological information data are divided by wavelet transform, data unnecessary as biological information and data having no effect on the characteristics of the biological information data are deleted, and the remaining biological information data are compressed. Then, it is judged whether the ECG data have been compressed at a prescribed compression ratio (STEP-3). When the ECG data have been compressed at the prescribed compression ratio, the data compression is completed and the compressed ECG data are output to the data transmitter 18. However, when the ECG data have not been compressed at the prescribed compression ratio, the conditions of data to be deleted are changed, data deletion is performed according to the changed conditions, and the remaining ECG data are recompressed (STEP-4). Then, the process of Step 3 is repeated on the recompressed ECG data until the ECG data are compressed at the prescribed compression ratio and the compressed ECG data are output to the data transmitter 18.

In this embodiment, the ECG data have been described. However, the invention can be applied to biological Information data on respiration, blood pressure, a pulse wave, etc. Also, it is possible that one or more kinds of biological data are compressed to achieve a desired data compression ratio and the acquired biological information data are transmitted.

Specific examples of the ECG data compression used by the data compressor 16 will be described below.

In an ECG data compression using Huffman coding, codes are assigned to the input ECG data such that the higher the frequency of occurrence of each data piece is, the smaller the number of bits of assigned code is, whereby the input ECG data are represented by a smaller amount of data than the original data. In this way, the ECG data are compressed.

In an ECG data compression using arithmetic coding, weighting are performed to pieces of input ECG data on the basis of the frequency of occurrence of each piece of the input ECG data and one number is calculated from the weighted ECG data and a plurality of values within a prescribed interval. In this way, the ECG data are compressed.

In an ECG data compression using wavelet transform, original data are filtered, and the components of the filtered data are divided into high-frequency components and low-frequency components. From the data generated at that time, data of frequency components unnecessary for exhibit characteristics as the ECG data or data having negligible effect on the characteristics is deleted, thereby reducing the amount of data. Then, the remaining ECG data is compressed. In this way, ECG data compressing is performed. In this case, when the data filtering is performed, it is possible to use a wavelet function, such as a Haar function, a Daubechies function, a bior 4.4 function, or a bior 6.8 function.

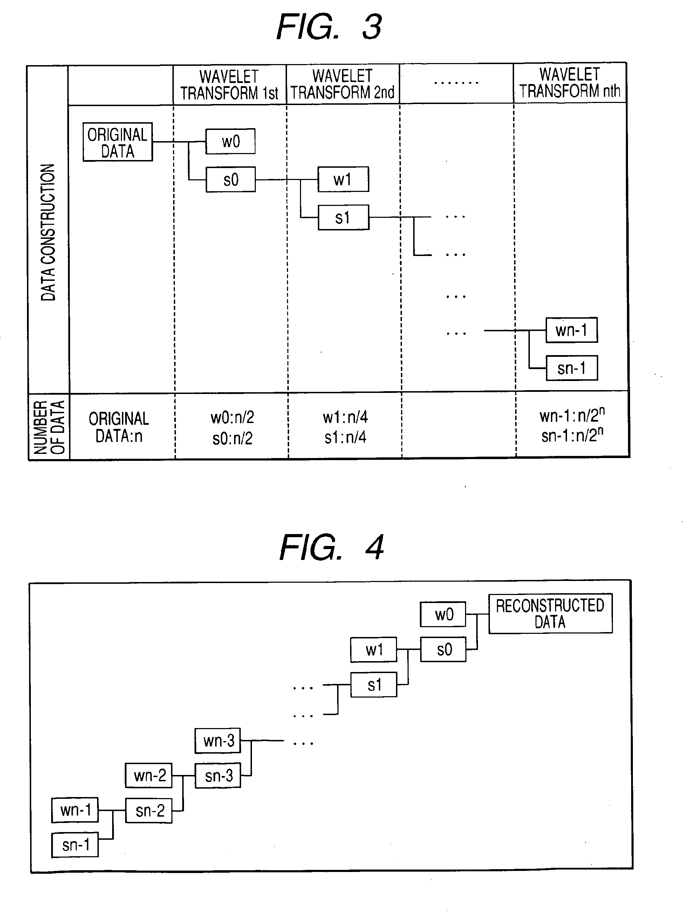

In the data compression using wavelet transform, it is confirmed that, as shown in Fig. 3, as the number of times wavelet transform is applied increases, the amount of data is reduced by half.

Subsequently, data necessary for reconstructing original data from data generated by the wavelet transform is data sn-1, wn-1, wn-2, wn-3, ..., w1, and w0. Data that is reconstructed by using all of the data are completely the same with the data before the wavelet transform (see Fig. 4). In this case, the amount of the reconstructed data is the same as the amount of the original data.

However, the data generated by the wavelet transform are divided according to the frequencies of the data. In respect to that, it is possible to reduce the amount of data by deleting data within an unnecessary frequency range or deleting a part of the data within a necessary frequency range so as not to damage a waveform, resulting in data compression. In this case, the data is reconstructed by considering the value of the deleted data as a specific value (for example, 0).

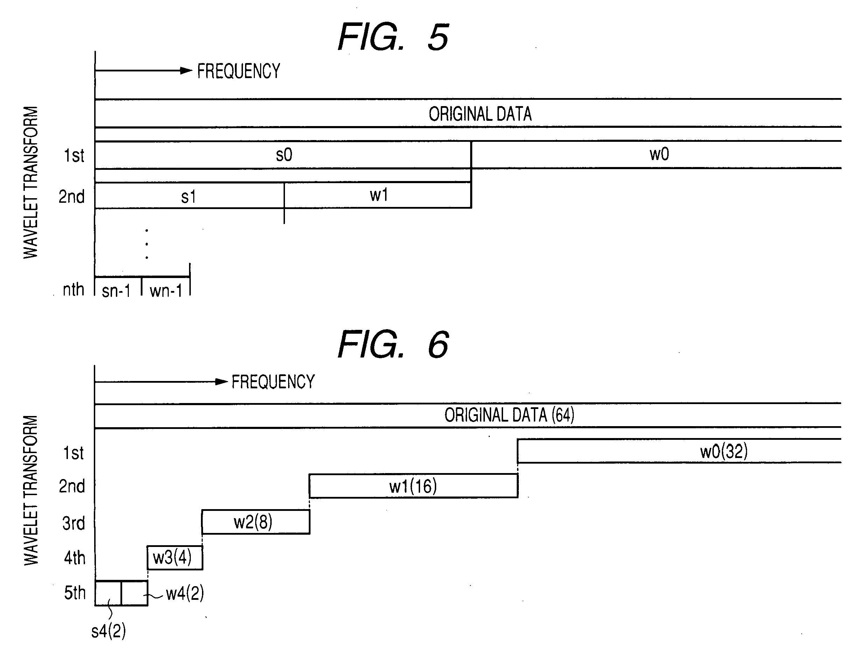

Fig. 5 shows the relationship between a frequency range and the number of times the wavelet transform is applied to the data. Fig. 6 shows the relationship between the amount of data and the frequency range when the wavelet transform is applied to 64 original data pieces five times.

As shown in Fig. 6, the original data have a wide frequency range and are divided into six frequency range w0 to w4 and s4. In this case, when a frequency component within the frequency range w0 is deletable, the number of data pieces required for reconstruction is reduced by 32. That is, the number of data pieces required for reconstruction is 32 which is a value obtained by subtracting 32 from 64. At this time point, the data compression ratio of 50% is achieved.

Further, in a case in which some of the data pieces has been deleted with respect to the frequency range w1 to w4 such that the characteristics of the waveform are not damaged, for example, when the number of data pieces within each of the frequency range w1 to w4 can be halved, the number of data pieces within each of the frequency range w1 to w4 is as follows: w1:16/2=8, w2:8/2=4, w3:4/2=2. w4:2/2=1. In this case, the total number of data pieces within the frequency range w0 to w4 and s0 becomes 17 (=0+8+4+2+1+2). As a result, the data compression ratio of 27% (=17164) is achieved.

When the above-mentioned operation is performed according to the characteristics of various waveforms, it is possible to perform data compression so as to achieve a desired number of data pieces.

A method of obtaining a reconstructed waveform when ECG data compression has been performed by using an ECG telemetry system having the above-mentioned structure and the data compression using the wavelet transform will be described.

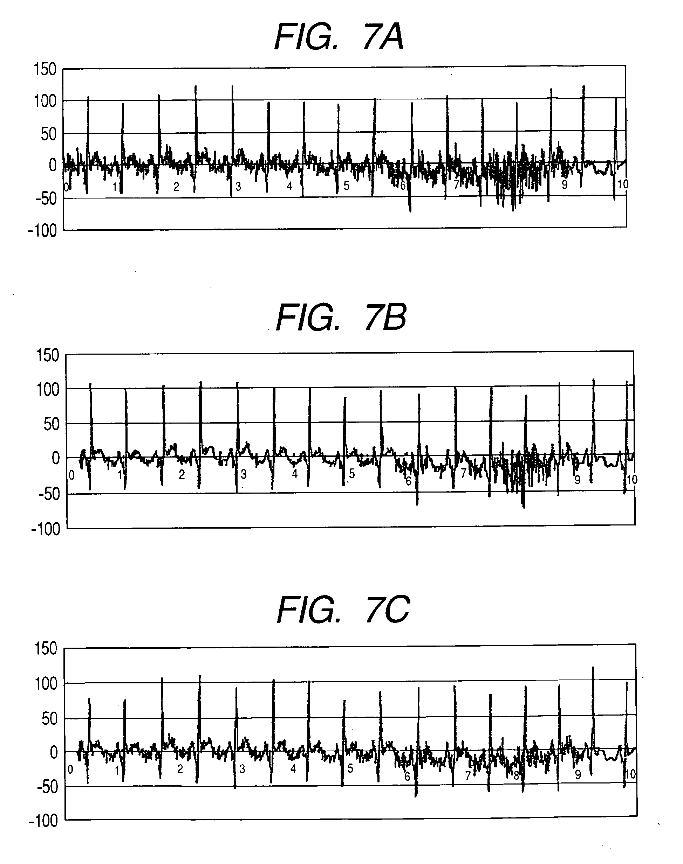

It was performed a test for determining a value to be deleted according to whether the height of a noise wave is large or small on the basis of the part of an ECG other than a QRS wave. Fig. 7A shows an ECG waveform on which noise has been superimposed. In the first half of the ECG, noise, at a level so as to make it difficult to distinguish the waves of the ECG other than the QRS wave, is superimposed, and in the second half of the ECG, noise, at a level so as to make it impossible to distinguish the waves of the ECG other than the QRS wave, is superimposed.

In this case, on the basis of whether it is possible to distinguish the waves other than QRS wave, it was determined that the wave height of the noise in the first half was small and the wave height of the noise in the second half was large.

Reconstructed waveforms in cases where a data compression is performed on the first and second halves are shown in Figs. 7B and 7C.

That is, as shown in Fig. 7B, when small values of the noise was deleted and then data compression was performed, it was possible to characteristically improve a part in which noise having a small wave height is mixed. Also, as shown in Fig. 7C, when large values of the noise was deleted and then data compression was performed, it was possible to characteristically improve a part in which noise having a large wave height is mixed. However, in each of the results shown in Figs. 7B and 7C, a part exhibiting the characteristics of the waveform of the ECG is rarely affected and thus the original characteristics of the waveform of the ECG are kept.

Therefore, in the ECG data compression using the wavelet transform, conditions for performing a data compression are as follows.
(1) Data exceeding several tens Hz, which is not important from frequency components of the ECG, are deleted from the frequency division data generated by the wavelet transform such that the number of data pieces is reduced, and then a data compression is performed.
(2) It becomes possible to perform data compression on the ECG while keeping the original characteristics of the waveform by deleting of all of the frequency data within a frequency range, deleting of small values including superimposed noise from the frequency division data, or a combination thereof.
(3) It becomes possible to perform data compression on the ECG while keeping the original characteristics of the waveform by deleting of all frequency data within a frequency range, deleting of large values including superimposed noise from the frequency division data, or a combination thereof.

Also, the amount of data is controlled by changing or combining the above-mentioned conditions (1) to (3), thereby variably setting the data compression ratio. When the data compression ratio is variably set, it is possible to properly perform data compression according to biological information data, such as ECG data, to be transmitted or the amount of the biological information data.

According to the above-mentioned ECG data compression and the ECG telemetry system executing the ECG data compression according to the embodiments of the invention, it is possible to attain the following advantageous effects.

When data to be essentially transmitted (for example, biological information) and data for interpolating the biological information are simultaneously transmitted with a prescribed communication bandwidth (data amount), it is possible to perform data compression until all of the data is fit into the prescribed amount of data. Then, a receiver receives the data and checks whether the data; such as biological information, are normal. When it is determined that the data is not normal, the data, such as biological information, which are not normal, is reproduced by using the interpolation data within the received data, thereby returning the data to normal data. Therefore, it is possible to obtain an ECG telemetry system capable of preventing lack of data.

When data to be essentially transmitted (for example, biological information) are repeatedly transmitted with a prescribed communication bandwidth (data amount), it is possible to perform data compression until all of the data is fit into the prescribed amount of data. Then, the receiver receives the data and checks whether the data, such as biological information, received first are normal. When it is determined that the data is not normal, it is possible to return the data, such as biological information, received first to normal data by using the data repeatedly received other than the data received first. Therefore, it is possible to obtain an ECG telemetry system capable of preventing data loss.

When a plurality of data pieces are simultaneously transmitted in a prescribed communication bandwidth (data amount), it is possible to perform data compression until all of the data is fit into the prescribed amount of data. Therefore, it is possible to obtain an ECG telemetry system in which a receiver can receive a plurality of data pieces, such as biological information.

When data, such as biological information, and data for detecting and correcting data error in a communication path are simultaneously transmitted in a prescribed communication bandwidth (data amount), it is possible to perform data compression until all of the data is fit into the prescribed amount of data. Then, a receiver receives the data and checks whether the data, such as biological information, are normal. When it is determined that the data is not normal, an error in data, such as biological information, is detected and corrected by using the data for detecting and correcting erroneous data, thereby restoring the data to normal data. Therefore, it is possible to obtain an ECG telemetry system having the above-mentioned function.

The ECG data compression and the ECG telemetry system according to the embodiments of the invention can be applied to other biological information, such as respiration, a blood pressure, an electroencephalogram, and a pulse wave, so as to delete a part of the data according to the characteristics of the biological information and perform a data compression.

Although only some exemplary embodiments of the invention have been described in detail above, those skilled in the art will readily appreciated that many modifications are possible in the exemplary embodiments without materially departing from the novel teachings and advantages of the invention. Accordingly, all such modifications are intended to be included within the scope of the invention.

For example, in the above embodiment, the data compression is performed with respect to waveforms on which noises are superposed. However, the data compression may be performed with respect to waveforms on which noises are not superposed by deleting frequency-divided data having a certain frequency range.

## Claims

1. A method of compressing an electrocardiogram data, comprising:
acquiring the electrocardiogram data;
subjecting the acquired electrocardiogram data to a frequency filtering; and
compressing the electrocardiogram data, which has been subjected to the frequency filtering, with either a wavelet transform, a Huffman coding, or an arithmetic coding.

2. The method as set forth in claim 1, wherein:
the wavelet transform includes dividing the electrocardiogram data into a plurality of data pieces which are respectively associated with different frequency ranges; and
the wavelet transform includes at least one of:
deleting at least one of the divided pieces;
deleting at least a part of at least one of the divided pieces, in which noises having a wave height less than a prescribed value are superposed; and
deleting at least a part of at least one of the divided pieces, in which noises having a wave height no less than the prescribed value are superposed.

3. An electrocardiogram telemeter, comprising:
a data acquirer, operable to acquire electrocardiogram data;
a filter, operable to subject the electrocardiogram data acquired by the data acquirer to a frequency filtering; and
a data compressor, operable to compress the electrocardiogram data, which has been subjected to the frequency filtering, with either a wavelet transform, a Huffman coding, or an arithmetic coding, thereby generating compressed electrocardiogram data adapted to be transmitted to a remote receiver which is configured to reconstruct the electrocardiogram data.

4. The electrocardiogram telemeter as set forth in claim 3, wherein:
the data compressor is operable to divide the electrocardiogram data into a plurality of data pieces which are respectively associated with different frequency ranges; and
the data compressor is operable to perform the wavelet transform by at least one of:
deleting at least one of the divided pieces;
deleting at least a part of at least one of the divided pieces, in which noises having a wave height less than a prescribed value are superposed; and
deleting at least a part of at least one of the divided pieces, in which noises having a wave height no less than the prescribed value are superposed.
